# EUROPEAN PATENT APPLICATION

(11) **EP 1 433 856 A1**
(43) Date of publication of application: **30.06.2004**
(21) Application number: 02799459.9
(22) Date of filing: 05.09.2002
(51) Int. Cl.: C12P 7/02, C12N 15/09

(54) **PROCESS FOR PRODUCING ALCOHOL WITH THE USE OF MICROORGANISM**

(30) Priority: 06.09.2001 JP 2001270903
(71) Applicant: Ajinomoto Co., Inc., Tokyo 104-8315 (JP)
(72) Inventor: TAKESHITA, Ryo, C/O AJINOMOTO Co., Inc., Kanagawa 210-868 1 (JP); YASUEDA, Hisashi, C/O AJINOMOTO Co., Inc., Kanagawa 210-8681 (JP); GUNJI, Yoshiya, C/O AJINOMOTO Co., Inc., Kanagawa 210-8681 (JP)
(74) Representative: HOFFMANN - EITLE
(86) International application number: PCT/JP2002/009029
(87) International publication number: WO 2003/027301

(57) **Abstract**

A recombinant of a microorganism that does not inherently utilize an alkane and an alcohol, which recombinant has acquired an ability to convert the alkane into the alcohol because of transformation with a DNA encoding a methane oxygenase, is cultured, and the obtained culture, cells isolated from the culture or processed product of the cells is allowed to exist with the alkane to produce the alcohol.

## Description

### Technical Field

The present invention relates to a method for producing an alcohol such as methanol by utilizing a microorganism. More specifically, the present invention relates to a method for converting an alkane into an alcohol under extremely mild conditions of biochemical oxidation using a microorganism.

### Background Art

The currently used process for producing methanol comprises a "synthesis gas production step" for reforming methane as the raw material into a mixed gas of carbon monoxide and hydrogen, and a "methanol synthesis step" for reacting the synthesis gas in the presence of a catalyst to convert the gas into methanol. The synthesis gas production step and the methanol synthesis step are carried out at a high temperature and high pressure, i.e., the synthesis gas production step is carried out at 850 to 880°C, and the methanol synthesis step is carried out at 50 to 100 atm. Therefore, a methanol synthesis method that can be carried out under milder conditions is desirable, and has been studied. Although a method of synthesizing methanol from methane with high yield by using a comparatively thermostable metal complex-type catalyst has been reported recently (Science, Vol. 280, 24, 560-563, April, 1998), this method still requires a temperature of 100° C or higher.

Meanwhile, if a methane monooxygenase enzyme (hereinafter abbreviated as "MMO") of a methane-utilizing bacterium is used, methanol can be directly synthesized from methane at an ordinary temperature and ordinary pressure. It is known that there is soluble-type MMO and a membrane-bound-type MMO. The soluble-type MMO enzyme is a complex protein consisting of three components: hydroxylase, Component B and reductase (Component C). The oxidation reaction is carried out by the hydroxylase among the components, and it has been reported that if this hydroxylase is chemically reduced instead of using a reductase, the oxidation reaction can be catalyzed by the hydroxylase alone (J. Biol. Chem., 264 (17) 10023-10033, 1989). The hydroxylase is also called Component A, and consists of three kinds of protein subunits, α, β and γ.

U.S. Patent No. 5,190,870 discloses an enzymatic method for producing an alkanol utilizing a hydroxylase purified from a methane-utilizing bacterium. However, the method for purifying this hydroxylase is complicated, and a decrease in the activity of the enzyme is significant after isolation thereof, which results in an unstable activity of the enzyme. Furthermore, although U.S. Patent No. 5,192,672 discloses a method for stabilizing an activity of hydroxylase after purification, the fundamental problem of the complicated purification method is still unsolved.

Furthermore, a method of microbiologically producing methanol using a methane-utilizing bacterium itself is also known. However, cells having MMO also have a methanol dehydrogenase etc., and therefore the problem exists that methanol produced by the oxidation of methane is immediately oxidized and thereby converted into formaldehyde, i.e., the methanol is metabolized in the cells. Japanese Patent Laid-open (KOKAI) No. 3-43090 discloses a method of selectively inhibiting a methanol dehydrogenase with cyclopropane. However, the method is extremely complicated, i.e., it comprises substitution of cyclopropane for aerial phase in a suspension of cells containing MMO, subsequent removal of the cyclopropane with helium etc., and the method also suffers from the problem that the methanol dehydrogenase may not be sufficiently inactivated with cyclopropane.

In the Journal of General Microbiology (1992), 138, 1301-1307, it is described that MMO activity could be successfully expressed by expressing Component B and reductase of the soluble-type MMO derived from *Methylococcus capsulatus* (Bath) in *Escherichia coli* and mixing them with a natural hydroxylase. Furthermore, in Arch. Microbiol. (1999) 171:364-370, it is reported that the MMO activity could be successfully expressed by introducing a gene of soluble-type MMO into a methane-utilizing bacterium having only the membrane-bound-type MMO. However, there is no example of expressing the activities of all of the components of MMO in a microorganism that does not utilize methane or methanol, and therefore, it is desirable to express the complicated enzyme complex in a heterogenous organism.

### Disclosure of the Invention

An object of the present invention is to express all of the components of soluble-type MMO (henceforth also abbreviated as "sMMO") in a microorganism that does not utilize methane and methanol and thereby produce active sMMO, and to provide a method for producing an alcohol from an alkane by utilizing such a microorganism.

The inventors of the present invention originally attempted to introduce a gene cluster encoding the soluble-type MMO into a microorganism which lacked any enzyme that oxidizes methanol such as methanol dehydrogenase, i.e., a microorganism that does not utilize methane or methanol, to construct a microorganism that expresses the gene cluster as active proteins and thereby provide a method for producing methanol from methane by utilizing the microorganism. However, sMMO is a complex protein consisting of 5 subunits, and therefore it is not easy to express it in an active form, in particular, in a heterogenous microorganism. Therefore, they conducted various research on types of plasmid and promoter used for a plasmid for expressing the soluble-type MMO and culture conditions. As a result, they succeeded in expressing sMMO in cells of a heterogenous microorganism while maintaining the activity thereof. Furthermore, they also succeeded in producing methanol from methane by using the obtained microorganism, and thus accomplished the present invention.

The present invention provides the followings.
(1) A method for producing an alcohol, comprising culturing a recombinant of a microorganism that does not inherently utilize an alkane and an alcohol which is generated by oxidation of the alkane, which recombinant has acquired an ability to convert the alkane into the alcohol due to transformation with a DNA encoding a methane oxygenase, and allowing the obtained culture, cells isolated from the culture or processed product of the cells to exist with the alkane to produce the alcohol.
(2) The method for producing an alcohol according to (1), wherein the methane oxygenase is a soluble-type methane oxygenase.
(3) The method for producing an alcohol according to (2), wherein the methane oxygenase consists of a methane hydroxylase, Component B and reductase.
(4) The method for producing an alcohol according to any one of (1) to (3), wherein the DNA encoding the methane oxygenase is a soluble-type methane oxygenase gene of *Methylococcus capsulatus.*
(5) The method for producing an alcohol according to any one of (1) to (4), wherein the microorganism is an *Escherichia* bacterium, coryneform bacterium or *Bacillus* bacterium.
(6) The method for producing an alcohol according to (5), wherein the microorganism is an *Escherichia* bacterium.
(7) The method for producing an alcohol according to (6), wherein the microorganism is cultured at 20 to 30°C.
(8) The method for producing an alcohol according to any one of (1) to (7), wherein the alkane is an alkane having between 1 to 8 carbon atoms, and the alcohol is an alcohol which is generated by oxidation of the alkane.
(9) The method for producing an alcohol according to (8), wherein the alkane is methane, and the alcohol is methanol.

### DESCRIPTION OF THE PREFERRED EMBODIMENTS

Hereinafter, the present invention will be explained in detail.

The microorganism used for the present invention is a microorganism which does not inherently utilize an alkane and an alcohol which is generated by oxidation of the alkane, but the microorganism has acquired an ability to convert the alkane into the alcohol because it has been transformed with a DNA encoding sMMO. As for the characteristic that the microorganism used for the present invention does not utilize an alcohol, whether the microorganism converts an alkane into an alcohol or not is not essential, whereas it is preferred that the microorganism should not utilize the alcohol for accumulation of the produced alcohol.

The microorganism which does not inherently utilize an alkane and an alcohol which is generated by oxidation of the alkane is not particularly limited so long as it is a microorganism that can acquire an ability to convert the alkane to the alcohol. Specific examples include *Escherichia* bacteria such as *Escherichia coli,* coryneform bacteria such as and *Brevibacterium lactofermentum* (*Corynebacterium glutamicum*), and *Bacillus* bacteria such as *Bacillus subtilis* and so forth.

The DNA encoding a methane oxygenase is preferably a DNA encoding a soluble-type MMO (sMMO). sMMO consists of Component A (methane hydroxylase), Component B and Component C (reductase). Component A consists of the subunits α, β and γ. Although these components may be separately introduced into the microorganism, they are preferably introduced by using a single vector containing a DNA encoding all of the components. Hereinafter, a gene cluster encoding all of the components of sMMO is referred to as a sMMO gene for convenience.

The sMMO gene can be obtained from a chromosomal DNA of a methane-utilizing bacterium, for example, the *Methylococcus capsulatus* NCIMB 11132 strain. This strain can be obtained from NCIMB (The National Collections of Industrial, Food and Marine Bacteria Ltd., 23 St. Machar Drive, Aberdeen AB2 1RY, Scotland, UK).

An example of a method for preparing a fragment containing the sMMO gene from *Methylococcus capsulatus* as a methane-utilizing bacillus is described below. However, such a fragment can also be obtained from other methane-utilizing bacteria in a similar manner.

First, the medium used for culturing *Methylococcus capsulatus* may be any medium in which the bacterium can sufficiently proliferate. An example of a preferred medium includes the medium of Whittenbury et al. (J. Gen. Microbiol., 61, 205-208, 1970). Any space in a culture vessel containing the medium is replaced with a mixed gas of methane and an oxygen-containing gas (air etc.), and *Methylococcus capsulatus* is inoculated into the medium in contact with the gas. *Methylococcus capsulatus* is an aerobic bacterium, and the culture may be performed at 20 to 50°C under an aerobic condition as batch culture or continuous culture.

A DNA fragment containing the sMMO gene can be separated and obtained by the hybridization method in the manner described below from a DNA library derived from chromosomes of a *Methylococcus capsulatus* strain using, as a probe, a DNA fragment containing a part of the sMMO gene obtained through PCR (polymerase chain reaction) using oligonucleotides prepared based on the known nucleotide sequence of the sMMO gene (GenBank Accession M90050 M32314 M58498 M58499, Stainthorpe, A.C., et al., Gene, 91 (1), 27-34, 1990, SEQ ID NO: 5) as primers and a chromosomal DNA of *Methylococcus capsulatus* as a template. The open reading frames contained in the sMMO gene are designated mmoX, mmoY, mmoB, mmoZ, OrfY and mmoC in this order from the 5' end.

The chromosomal DNA can be extracted from culture broth of the *Methylococcus capsulatus* NCIMB 11132 strain by a usually used method known per se (e.g., the method described in Biochem. Biophys. Acta., 72, 619 (1963) etc.).

The DNA library can be prepared by decomposing the chromosomal DNA using a suitable restriction enzyme such as *BamHI,* ligating the obtained DNA fragments of various sizes with a plasmid vector such as pUC18 (produced by Takara Shuzo) and transforming a suitable host such as *Escherichia coli* JM109 with the ligation reaction mixture.

The probe for selecting a clone having a DNA fragment containing the sMMO gene by hybridization can be obtained by PCR using oligonucleotides suitably designed based on the known nucleotide sequence of the sMMO gene, for example, the nucleotide sequences shown as SEQ ID NOS: 1 and 2, as primers and a chromosomal DNA of *Methylococcus capsulatus* as a template.

Colony hybridization is performed for a chromosomal DNA library of the *Methylococcus capsulatus* NCIMB 11132 strain by using the probe obtained as described above. A DNA fragment containing the sMMO gene or a part thereof can be obtained by extracting a plasmid DNA from a clone that hybridized with the partial DNA fragment of the sMMO gene used as the probe in the hybridization and obtaining the inserted fragment through digestion of the plasmid DNA with a suitable restriction enzyme.

When the fragment of the clone obtained as described above contains a part of the sMMO gene, the other region can be obtained by the PCR method, hybridization method or the like. For example, when the cloned fragment does not have the 5' side region of the sMMO gene, the upstream region of the cloned fragment can be obtained by the 5'-RACE method. Moreover, the upstream region of the sMMO gene can also be obtained by PCR using the oligonucleotides shown as SEQ ID NOS: 3 and 4 as primers and the chromosomal DNA of the NCIMB 11132 strain as a template. The obtained DNA fragment can be ligated to the previously obtained sMMO gene fragment to obtain the full length sMMO.
Other than wild-type proteins, each component or subunit of sMMO may have an amino acid sequence which includes substitution, deletion, insertion or addition of one or several amino acid residues, as long as the function of each component or subunit is not degraded. Although the number of "several" amino acids referred to herein differs depending on position of amino acid residues in a three-dimensional structure of a protein or type of amino acids, it may be preferably between 2 to 10, more preferably between 2 to 5, most between preferably 2 to 3.

The DNA encoding a protein or peptide substantially identical to the sMMO described above include a DNA that is hybridizable with an open reading frame in the nucleotide sequence shown as SEQ ID NO: 4 or a probe that can be produced from the nucleotide sequence under stringent conditions and codes for a protein that can constitute an active sMMO. The aforementioned "stringent conditions" include conditions under which a so-called specific hybrid is formed, and a non-specific hybrid is not formed. It is difficult to clearly express this condition by using any numerical value. However, for example, the stringent conditions include conditions under which DNAs having high homology, for example, DNAs having homology of 50% or more hybridize with each other, but DNAs having homology lower than the above do not hybridize with each other. Alternatively, the stringent conditions include conditions whereby DNAs hybridize with each other at a salt concentration corresponding to a typical washing conditions of Southern hybridization, i.e., 1 x SSC, 0.1% SDS, preferably 0.1 x SSC, 0.1% SDS, at 60°C.

The vector used for introducing the sMMO gene into a host microorganism may be any vector autonomously replicable in a cell of the host microorganism, and specific examples include, for *Escherichia coli,* plasmid vectors pUC19, pUC18, pBR322, pHSG299, pHSG298, pHSG399, pHSG398, RSF1010, pMW119, pMW118, pMW219, pMW218 and so forth. Furthermore, vectors for coryneform bacteria include pAM330 (refer to Japanese Patent Laid-open Publication No. 58-67699), pHM1519 (refer to Japanese Patent Laid-open Publication No. 58-77895), pAJ655, pAJ611, pAJ1844 (refer to Japanese Patent Laid-open No. 58-192900 for these), pCG1 (refer to Japanese Patent Laid-open No. 57-134500), pCG2 (refer to Japanese Patent Laid-open No. 58-35197), pCG4, pCG11 (refer to Japanese Patent Laid-open No. 57-183799 for these), pHK4 (refer to Japanese Patent. Laid-open No. 5-7491) and so forth. Furthermore, vectors for *Bacillus* bacteria include pUB110, pHY300PLK, pHV1248, pE194, pC194, pBC16, pSA0501, pSA2100, pAM77, pT181, pBD6, pBD8 and pBD64, pHV14 and so forth.

The promoter of the sMMO gene may be replaced with a suitable promoter depending on the microorganism into which the gene is introduced. Examples of such promoters include lac promoter, trp promoter, trc promoter, tac promoter, P_{R} promoter and P_{L} promoter of lambda phage, tet promoter, amyE promoter and so forth. If an expression vector containing a promoter is used as the vector, ligation of the sMMO gene, vector and promoter can be attained by a single ligation operation. Examples of such vectors include pKK233-3 containing the tac promoter (produced by Pharmacia) and so forth.

As the transformation method for introducing a vector incorporated with the sMMO gene into an objective microorganism include, for example, a method of treating recipient cells with calcium chloride so as to increase the permeability of the cells for DNA, which has been reported for *Escherichia coli* K-12 (Mandel, M. and Higa, A., J. Mol. Biol., 53, 159 (1970)), and a method of preparing competent cells from cells which are at the growth phase followed by introducing the DNA thereinto, which has been reported for *Bacillus subtilis* (Duncan, C.H., Wilson, G.A. and Young, F.E., Gene, 1, 153 (1977)) can be used. Alternatively, a method of making DNA-recipient cells into protoplasts or spheroplasts, which can easily take up recombinant DNA, followed by introducing the recombinant DNA into the DNA-acceptor cells, which is known to be applicable to *Bacillus subtilis,* actinomycetes and yeasts (Chang, S. and Choen, S.N., Molec. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Sci., USA, 75, 1929 (1978)) can also be applicable. The transformation method can be appropriately selected from these methods depending on the cell used as the host. Furthermore, the recombinant DNA can be introduced into a recipient bacterium belonging to *Brevibacterium* or *Corynebacterium* bacteria by the electroporation method (Sugimoto et al., Japanese Patent Laid-open No. 2-207791) (OP199).

Methods for preparation of genomic DNA library, hybridization, PCR, preparation of plasmid DNA, digestion and ligation of DNA, transformation etc. are described in Sambrook, J., Fritsch, E.F., Maniatis, T., Molecular Cloning, Cold Spring Harbor Laboratory Press, 1.21 (1989).

The *E. coli* JM109 strain transformed with an expression vector pRSsMMOTB containing the sMMO gene obtained as described above was deposited at the independent administrative agency, National Institute of Advanced Industrial Science and Technology, International Patent Organism Depository (postal code 305-5466, Tsukuba Central 6, 1-1 Higashi 1-Chome, Tsukuba-shi, Ibaraki-ken, Japan) on August 2, 2001 and given an accession number of PERM P-18446. Then, the deposition was converted into an international deposition under the provisions of the Budapest Treaty on August 19, 2002 and given with an accession number of FERM BP-8153.

By culturing a microorganism which acquired an ability to convert an alkane into an alcohol by transformation with a DNA encoding a DNA containing the sMMO gene and allowing the obtained culture or cells isolated from the culture to exist with the alkane to produce the alcohol, the alcohol can be produced.

The medium used for the culture of the microorganism introduced with the sMMO gene may be appropriately selected depending on the microorganism used. For example, it may be a typical medium that contains a carbon source, nitrogen source, inorganic ions, and other organic ingredients as required.

As the carbon source, there can be used saccharides such as glucose, sucrose, lactose, galactose, fructose or starch hydrolysate, alcohols such as glycerol or sorbitol, or organic acids such as fumaric acid, citric acid or succinic acid.

As the nitrogen source, there can be used inorganic ammonium salts such as ammonium sulfate, ammonium chloride or ammonium phosphate, organic nitrogen such as soybean protein hydrolysate, ammonia gas, aqueous ammonia and so forth.

It is desirable to add required substances such as vitamin B₁, yeast extract and so forth to the medium in appropriate amounts as organic trace nutrients. Other than the above, potassium phosphate, magnesium sulfate, iron ions, manganese ions and so forth are added in small amounts as required.

The culture is preferably carried out under an aerobic condition for 16 to 72 hours. The culture temperature is preferably controlled to be 25°C to 45°C, and pH is preferably controlled to be 5 to 8 during the culture. Inorganic or organic, acidic or alkaline substances as well as ammonia gas and so forth can be used for adjustment of pH.

When an *Escherichia* bacterium such as *Escherichia coli* is used as the host microorganism, the culture is preferably performed at 20 to 30°C in order to produce the subunits constituting sMMO as soluble peptides.

As the catalyst for conversion of an alkane to an alcohol, besides cells and culture containing cells, a processed product of the cells may also be used. The processed product of cells may be cells treated with acetone, lyophilized cells, cell-free extract prepared from the acetone-treated or lyophilized cells or live cells, fractionation product such as membrane fraction fractionated from the cell-free extract, and immobilized product of the cells, cell-free extract or fractionation product. By bringing such cells or processed product of cells into contact with an alkane and allowing a reaction, an alcohol can be produced in the reaction solution. The microorganism used may consist of one kind of microorganism or a mixture of two or more kinds of arbitrary microorganisms. If the alkane does not inhibit growth of the microorganism, the alkane can be added to the medium used for the culture of the microorganism to simultaneously perform the culture of the microorganism and production of the alcohol. In such a case, the alkane can be introduced into the medium by adding it into a gas phase in contact with the medium or bubbling the medium with the alkane. Furthermore, by using a microorganism exhibiting strong reducing power such as *Escherichia* bacteria as the microorganism used for the present invention or by adding NADH to the reaction mixture, the reaction catalyzed by sMMO can be efficiently advanced.

The alkane to which the present invention is applied is preferably an alkane having between 1 to 8 carbon atoms, more preferably 1 to 6 carbon atoms, particularly preferably 1 to 5 carbon atoms. Especially, methane is the most preferred. Furthermore, the position of hydrogen atom in the alkane oxidized by sMMO is not particularly limited, and it may be hydrogen binding to an end carbon or hydrogen binding to a carbon to which two or more carbons bind. The alkane may be a linear, branched or cyclic alkane. The alkane may have a substituent such as a halogen.

The substrate specificity of sMMO derived from the *Methylococcus capsulatus* Bath strain has been studied, and it has been revealed that the strain has an ability to oxidizing alkanes, including at least methane to octane to produce an alcohol.

### Brief Explanation of the Drawing

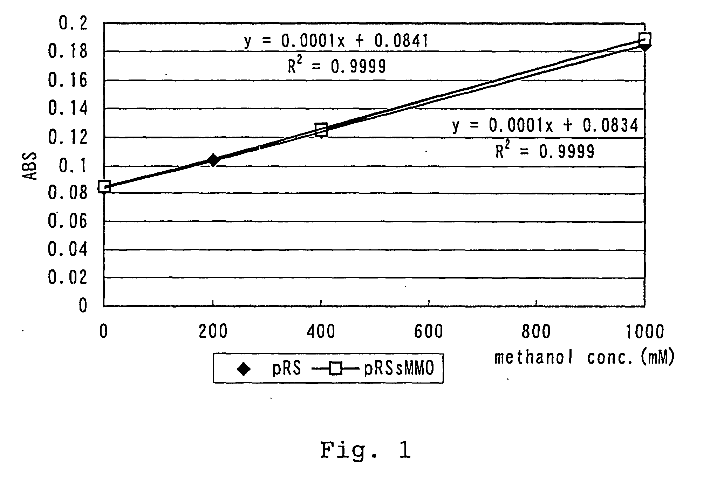

Fig. 1 shows calibration curves prepared by plotting the methanol concentration (mM) in abscissa and the absorbance in ordinate. The results for *E. coli* JM109/pRS are indicated with **◆,** and the results for *E. coli* JM109/pRSsMMOTB (AJ13852) are indicated with ■.

### Best Mode for Carrying out the Invention

Hereinafter, the present invention will be explained more specifically with reference to the following examples.

### <1> Preparation of chromosomal DNA library of methane-utilizing bacterium, Methylococcus capsulatus

A space in a culture vessel containing the medium of Whittenbury et al. (J. Gen. Microbiol., 61, 205-208, 1970) was replaced with a mixed gas of methane and air. The methane-utilizing bacterium, *Methylococcus capsulatus* NCIMB 11132 strain, was inoculated into the medium in contact with the gas and cultured under aerobic conditions as batch culture while gas replacement was continued.

Chromosomal DNAs were extracted from the cells of the *Methylococcus capsulatus* NCIMB 11132 cultured as described above by the method described in Biochem. Biophys. Acta., 72, 619 (1963). The chromosomal DNAs were fully digested by using the restriction enzyme *BamHI.* The obtained DNA fragments of various sizes were inserted into a plasmid vector pUC18 (produced by Takara Shuzo) at the *BamHI* site. *Escherichia coli* JM109 was transformed with the obtained recombinant plasmids to prepare a chromosomal DNA library.

### <2> Cloning of sMMO gene by colony hybridization

A clone containing a sMMO gene fragment was selected from the aforementioned chromosomal DNA library by colony hybridization. A hybridization probe was prepared by amplifying the sMMO gene fragment by the PCR method. The nucleotide sequence of the sMMO gene of *Methylococcus capsulatus* was has been reported (Gene, 91, 27-34 (1990)), and the oligonucleotides having the nucleotide sequences shown as SEQ ID NOS: 1 and 2 were synthesized based on that sequence.

The chromosomal DNAs of *Methylococcus capsulatus* prepared as described above was used as the template, and the aforementioned oligonucleotides were used as primers to perform PCR. For PCR, a cycle consisting of a denaturation step (94°C, 10 seconds), an annealing step (55°C, 30 seconds) and an extension step (72°C, 1 minute and 30 seconds) were performed for 30 cycles.

Colony hybridization was performed for the aforementioned chromosomal DNA library by using the partial fragment of the sMMO gene amplified as described above as a probe. Labeling of the probe and the hybridization reaction were performed by using DIG-High Prime DNA Labeling & Detection Kit I (purchased from Boehringer Mannheim) according to the attached protocol.

Recombinant plasmid DNAs were extracted from the clones that showed positive results for hybridization, and the plasmid DNAs were digested with the restriction enzyme *BamHI* to confirm the inserted fragments. As a result, in addition to a DNA fragment having a length of about 2.3 kb corresponding to the length of the plasmid pUC18, an inserted DNA fragment having a size of about 6 kb was confirmed.

This inserted DNA was digested with various restriction enzymes to confirm that it was a fragment containing a large part of the objective sMMO gene. As a result, it became clear that a part of a gene encoding the α subunit constituting sMMO was deleted in the aforementioned fragment. The recombinant plasmid containing this inserted DNA fragment of about 6 kbp was designated pUC6K.

### <3> Construction of plasmid containing sMMO gene of full length

Then, an upstream region of the sMMO gene including deletion was obtained by PCR as described below. The nucleotide sequences of the synthesized primers are shown in Sequence Listing as SEQ ID NO: 3 and 4. The chromosomal DNAs of the NCIMB 11132 strain were used as a template together with the aforementioned oligonucleotides as primers to perform PCR. For PCR, a cycle consisting of a denaturation step (98°C, 10 seconds), an annealing step (60°C, 30 seconds) and an extension step (72°C, 180 seconds) were performed for 30 cycles. The obtained amplified DNA fragment of about 1.5 kbp was digested with the restriction enzymes *EcoRI* and *BamHI* and ligated to the multi-cloning site of pUC118. A fragment of about 6 kbp excised from pUC6K with *BamHI* was incorporated into the *BamHI* site of the aforementioned plasmid to obtain a plasmid pUCsMMO containing the sMMO gene of the full length.

### <4> Construction of sMMO gene expression plasmid

Then, the sMMO gene region was excised from the aforementioned plasmid pUCsMMO using the restriction enzymes *EcoRI* and *HindIII* and incorporated into an expression vector pKK233-3 (produced by Pharmacia) downstream from the tac promoter to obtain an expression plasmid pKKsMMO which was constructed so that transcription from the tac promoter should proceed to the sMMO gene.

The *smmo* gene including the tac promoter region derived from pKK3-233 was excised from pKKsMMO by digestion with the restriction enzymes *NdeI* and *DraI,* blunt-ended, digested with the restriction enzyme *PstI,* and then ligated to a blunt-ended broad host spectrum vector pRS (described in International Patent Publication in Japanese (Kohyo) No. 3-501682) to obtain a sMMO expression plasmid pRSsMMOTB.

### <5> Confirmation of expression of sMMO

The AJ13852 strain was inoculated into the LB liquid medium containing 20 mg/ml of streptomycin, precultured overnight at 25°C, then inoculated into a similarly prepared liquid medium in an amount of 1% (v/v), and cultured at 25°C as the main culture. During the main culture, when OD660 reached 0.6, IPTG (isopropyl-β-D-thiogalactopyranoside) was added to the medium at a final concentration of 1 mM, and the cells were further cultured for 2.5 hours. The cells obtained with the aforementioned culture conditions were disrupted by ultrasonication and fractionated into a soluble fraction and insoluble fraction by centrifugation. Each fraction was analyzed by using antibodies directed to each of the component peptides constituting sMMO. As a result, it was confirmed that all of the subunits, α, β and γ and both of Components B and C of sMMO existed in the soluble fraction.

When all of the aforementioned culture steps were performed at 37°C, the α subunit completely formed inclusion bodies and did not exist in the soluble fraction. Furthermore, most of the β and γ subunits were also insoluble. Moreover, when the culture was performed at 30°C, the amount of the α subunit existing in the soluble fraction became smaller as compared with the case where the culture was performed at 25°C.

### <6> Production of methanol from methane

The AJ13852 strain was cultured in the same manner as described above by using 100 ml of the medium (temperature was 25°C), and the cells were collected and then washed with 50 mM potassium phosphate buffer (pH 7.0). Then, the cells were suspended in the same buffer (pH 7.0), and a cell-free extract was obtained by ultrasonication. The total protein concentration of the cell-free extract was adjusted to 15 mg/ml. In a volume of 1 ml of the aforementioned cell-free extract was introduced into a 5-ml volume aluminum seal vial, mixed with NADH (reduced nicotinamide adenine dinucleotide) to a final concentration of 1 mM and sealed. Then, 5 ml (volume under 1 atm) of methane was enclosed in the vial, and a reaction was allowed. The reaction was allowed at 37°C for 60 minutes with shaking. Furthermore, the AJ13852 strain and *E. coli* JM109/pRS introduced only with the vector pRS were also cultured in the same manner as described above except that methane was not enclosed.

The methanol concentration was quantified using a quantification system having three steps of enzymatic reactions. The compositions of reaction mixtures are shown in Table 1. First, 600 µl of the reaction mixture after the aforementioned methanol formation reaction was taken, mixed with 50 µl of 5 N potassium hydroxide, sufficiently stirred and left for 5 minutes. Then, the reaction mixture was mixed with 50 µl of 5 M sodium chloride and adjusted to pH 7.5 with 1 M potassium phosphate buffer. Thereafter, each reaction mixture was used for the following enzymatic reaction after denatured proteins were removed by centrifugation.

**Table 1**

| Calibration curve | |
|---|---|
| MeOH (0 to 1000 mM) | 20 µl |
| Oxidized nicotinamide adenine dinucleotide (final | 2 µl |
| concentration: 1 mM) | |
| Alcohol oxidase (1 mg/ml) | 7 µl |
| Formaldehyde dehydrogenase (1 mg/ml) | 17 µl |
| Diaphorase (final concentration: 2 U/ml) | 20 µl |
| Iodonitrotetrazolium (final concentration: 1 mM) | 20 µl |
| Sample (without methane gas) | 20 µl |
| 25 mM Potassium phosphate buffer | 94 µl |
| | Total 200 µl |

| Measurement sample | |
|---|---|
| Oxidized nicotinamide adenine dinucleotide (final | 2 µl |
| concentration: 1 mM) | |
| Alcohol oxidase (1 mg/ml) | 7 µl |
| Formaldehyde dehydrogenase (1 mg/ml) | 17 µl |
| Diaphorase (final concentration: 2 U/ml) | 20 µl |
| Todonitrotetrazolium (final concentration: 1 mM) | 20 µl |
| Sample (with methane gas) | 20 µl |
| 25 mM Potassium phosphate buffer | 114 µl |
| | Total 200 µl |

The enzymatic reaction was performed according to the following procedures. Methanol was converted into formaldehyde with the alcohol oxidase, and formic acid was produced from formaldehyde with the formaldehyde dehydrogenase. NADH produced in the above reaction was quantified using the color reaction of the diaphorase as absorbance at 550 nm. The alcohol oxidase and formaldehyde dehydrogenase were purchased from Sigma, diaphorase was purchased from Toyobo, and iodonitrotetrazolium was purchased from Nakalai Tesque.

The reaction mixtures obtained using the AJ13852 strain and *E. coli* JM109/pRS which was introduced only with the vector pRS without enclosing methane were subjected to the aforementioned three-step enzymatic reaction, and the measured absorbance values were considered to correspond to a methanol concentration of 0 M. On the basis of these values, methanol was added to the aforementioned quantification system at various concentrations, and the methanol amounts were plotted against absorbance values to prepare calibration curves (Fig. 1). As a result, it was found that the methanol concentration value linearly corresponded to the absorbance value in the methanol concentration range of 200 to 1000 µM in this measurement system.

The methanol production amount for each kind of cell was quantified using the aforementioned calibration curves. As a result, production of methanol was not detected for *E. coli* JM109/pRS, whereas production of methanol was detected for the AJ13852 strain, and the concentration was 240 µM. The specific activity in this reaction was 0.33 nmol/minute/1 mg protein in the enzyme solution.

### Industrial Applicability

According to the present invention, an ability to convert an alkane into an alcohol can be imparted to a microorganism which does not inherently utilize the alkane, and an alcohol which is generated by oxidation of the alkane, and the alcohol can be produced from the alkane using the obtained microorganism.

## Claims

1. A method for producing an alcohol, comprising culturing a recombinant of a microorganism that does not inherently utilize an alkane, and an alcohol which is generated by oxidation of an alkane, whereby said recombinant has acquired an ability to convert the alkane into the alcohol due to transformation with a DNA encoding a methane oxygenase, and allowing the obtained culture, cells isolated from the culture, or processed product of said cells to exist with the alkane to produce the alcohol.

2. The method for producing an alcohol according to claim 1, wherein said methane oxygenase is a soluble-type methane oxygenase.

3. The method for producing an alcohol according to claim 2, wherein said methane oxygenase consists of a methane hydroxylase, Component B and reductase.

4. The method for producing an alcohol according to any one of claims 1 to 3, wherein said DNA encoding the methane oxygenase is a soluble-type methane oxygenase gene of *Methylococcus capsulatus.*

5. The method for producing an alcohol according to any one of claims 1 to 4, wherein said microorganism is an *Escherichia* bacterium, coryneform bacterium or *Bacillus* bacterium.

6. The method for producing an alcohol according to claim 5, wherein said microorganism is an *Escherichia* bacterium.

7. The method for producing an alcohol according to claim 6, wherein said microorganism is cultured at 20 to 30° C.

8. The method for producing an alcohol according to any one of claims 1 to 7, wherein said alkane is an alkane having between 1 to 8 carbon atoms, and said alcohol is an alcohol which is generated by oxidation of the alkane.

9. The method for producing an alcohol according to claim 8, wherein said alkane is methane, and said alcohol is methanol.


SEQUENCE LISTING

<110> Ajinomoto Co., Inc.

<120> METHOD OF PRODUCING ALCOHOL UTILIZING MICROORGANISM

<130> C035SMOP1387

<140>
<141> 2002-09-06

<150> JP 2001-270903
<151> 2001-09-06

<160> 5

<170> PatentIn Ver. 2.0
<210> 1
<211> 38
<212> DNA
<213> Artificial Sequence

<220>
<223> Description of Artificial Sequence:primer

<400> 1
ggtaagttta tgcagcgagt tcacactatc acggcggt 38

<210> 2
<211> 30
<212> DNA
<213> Artificial Sequence

<220>
<223> Description of Artificial Sequence:primer

<400> 2

tcgaagcttc cccggttcag gccgccccgg                                    30

<210> 3
<211> 39
<212> DNA
<213> Artificial Sequence

<220>
<223> Description of Artificial Sequence:primer

<400> 3
cgtgaattcc cgtcggagca ttcggataac gtgctcatc                           39

<210> 4
<211> 30
<212> DNA
<213> Artificial Sequence

<220>
<223> Description of Artificial Sequence:primer

<400> 4
attaagctta agcgtgatag tcttcgagct tgcggtccag g                        41

<210> 5
<211> 7188
<212> DNA
<213> Methylococcus capsulatus

<220>
<221> CDS
<222> (1478)..(3061)

<220>
<221> CDS
<222> (3142)..(4311)

<220>

<221> CDS
<222> (4328)..(4753)

<220>
<221> CDS
<222> (4764)..(5276)

<220>
<221> CDS
<222> (5432)..(5743)

<220>
<221> CDS
<222> (5756)..(6802)

<400> 5
cggatcgttg ccggcttttc ccatcaggcc tcccgagcgg aaatggggaa gaggcggacc 60
tccgtcccca ccgcctcggc cagtacacgg cgagagaagt gctcgatcgc ggcggtgtcg 120
gcttcctgca ggatgtcgat cttgttcaac accaaaaaga tacggccggc gtacttccgg 180
acctctcatg gaggaactcc agttcggcct cactcaccgc ctggtcggcg gagaggacga 240
acagggcggc atcgcactgc ggcacgcgcg cccaggcggt gtcggtgttg tggcggaaca 300
ccgagccgac gccgggtgtg tccacgatgc gcacgccatt ccccaggagc ggcgagggcg 360
tccggatcag acttcccgca cgcccttcgc gttgcccggg ttgcccggct cggtgatgta 420
atccgcgagc atatccggac ggatgtcgaa ggggctgcct tccagcggct ggacggtgat 480
ccggggggga tagccgtagg cgagcacggt caccaccgag gtcagcggca ccgcccgcgt 540
gggcagcagg ttctcaccca gcagcgcgtt gatcaggctg gtcttgcccc gtttgaactg 600
gccgaccgtc aggacgtcga aatggccggc ctgcagcttg tcacgcaggg cgagggcggg 660
gcgcagagcg gcggggaggc gtccgccagc tcgtcgatca ggctcaggag ttcggttttg 720
agatccgcat agccggtcgt tggcgcctca ccggcgatcg gccaacgggg gatcgcgttc 780
attccgcagt ggtcggatcg atgacggtgc ggatttcgtt gatcgcatcg gcgggaaagc 840
cgccttccag tgcgtgctgt accagcgtgg cggcgtcggg ggcaatgtag acgcagtaca 900
ccttgtcgtc ggtgacatag ctctcgatcc actgaacctg cggtcccatg gcgttgagga 960
tcgaacagga ttacgggaga tctgccgcag ttcgtcgcgc gacaggtttc cggcggccgc 1020
gaatcgtgcg ctctatgaca tatttgggca tggttatcag ccttacggtt ctggtaagga 1080
aaaaataggc ttgtattgtg cttatccgaa gataagcgct ttccgcgcag cccgattctt 1140
tcatggatca cgattccatt gaatgcggcg aaagtctcag ggtccggtca tgaatgaaga 1200
gttatggcgg cccagtacgt caccgttatg tccgatggct gtatcaaaca aagacacgtg 1260
tagtgatatc ggacaactcg tccatccccg tcggagcatt cggataacgt gctcatcgtt 1320

ccaaaatatt gatatacggt atacgtatcc gaagaataaa gttggcacga tccctgtaac 1380
taggttgtca cgacctcgtc ggaggttgta tgtccggtgt tccgtgacgt catcgggcat 1440
tcatcattca tagaatgtgt tacggaggaa acaagta atg gca ctt agc acc gca 1495
Met Ala Leu Ser Thr Ala
1               5
acc aag gcc gcg acg gac gcg ctg gct gcc aat cgg gca ccc acc agc 1543
Thr Lys Ala Ala Thr Asp Ala Leu Ala Ala Asn Arg Ala Pro Thr Ser
            10                  15                  20
gtg aat gca cag gaa gtg cac cgt tgg ctc cag agc ttc aac tgg gat 1591
Val Asn Ala Gln Glu Val His Arg Trp Leu Gln Ser Phe Asn Trp Asp
        25                  30                  35
ttc aag aac aac cgg acc aag tac gcc acc aag tac aag atg gcg aac 1639
Phe Lys Asn Asn Arg Thr Lys Tyr Ala Thr Lys Tyr Lys Met Ala Asn
    40                  45                  50
gag acc aag gaa cag ttc aag ctg atc gcc aag gaa tat gcg cgc atg 1687
Glu Thr Lys Glu Gln Phe Lys Leu Ile Ala Lys Glu Tyr Ala Arg Met
55                  60                  65                  70
gag gca gtc aag gac gaa agg cag ttc ggt agc ctg cag gat gcg ctg 1735
Glu Ala Val Lys Asp Glu Arg Gln Phe Gly Ser Leu Gln Asp Ala Leu
                75                  80                  85
acc cgc ctc aac gcc ggt gtt cgc gtt cat ccg aag tgg aac gag acc 1783
Thr Arg Leu Asn Ala Gly Val Arg Val His Pro Lys Trp Asn Glu Thr
            90                  95                  100
atg aaa gtg gtt tcg aac ttc ctg gaa gtg ggc gaa tac aac gcc atc 1831
Met Lys Val Val Ser Asn Phe Leu Glu Val Gly Glu Tyr Asn Ala Ile
        105                 110                 115
gcc gct acc ggg atg ctg tgg gat tcc gcc cag gcg gcg gaa cag aag 1879
Ala Ala Thr Gly Met Leu Trp Asp Ser Ala Gln Ala Ala Glu Gln Lys
    120                 125                 130
aac ggc tat ctg gcc cag gtg ttg gat gaa atc cgc cac acc cac cag 1927
Asn Gly Tyr Leu Ala Gln Val Leu Asp Glu Ile Arg His Thr His Gln
135                 140                 145                 150
tgt gcc tac gtc aac tac tac ttc gcg aag aac ggc cag gac ccg gcc 1975
Cys Ala Tyr Val Asn Tyr Tyr Phe Ala Lys Asn Gly Gln Asp Pro Ala
                155                 160                 165
ggt cac aac gat gct cgc cgc acc cgt acc atc ggt ccg ctg tgg aag 2023
Gly His Asn Asp Ala Arg Arg Thr Arg Thr Ile Gly Pro Leu Trp Lys
            170                 175                 180

ggc atg aag cgc gtg ttt tcc gac ggc ttc att tcc ggc gac gcc gtg 2071
Gly Met Lys Arg Val Phe Ser Asp Gly Phe Ile Ser Gly Asp Ala Val
        185                 190                 195
gaa tgc tcc ctc aac ctg cag ctg gtg ggt gag gcc tgc ttc acc aat 2119
Glu Cys Ser Leu Asn Leu Gln Leu Val Gly Glu Ala Cys Phe Thr Asn
    200                 205                 210
ccg ctg atc gtc gca gtg acc gaa tgg gct gcc gcc aac ggc gat gaa 2167
Pro Leu Ile Val Ala Val Thr Glu Trp Ala Ala Ala Asn Gly Asp Glu
215                 220                 225                 230
atc acc ccg acg gtg ttc ctg tcg atc gag acc gac gaa ctg cgc cac 2215
Ile Thr Pro Thr Val Phe Leu Ser Ile Glu Thr Asp Glu Leu Arg His
                235                 240                 245
atg gcc aac ggt tac cag acc gtc gtt tcc atc gcc aac gat ccg gct 2263
Met Ala Asn Gly Tyr Gln Thr Val Val Ser Ile Ala Asn Asp Pro Ala
            250                 255                 260
tcc gcc aag tat ctc aac acg gac ctg aac aac gcc ttc tgg acc cag 2311
Ser Ala Lys Tyr Leu Asn Thr Asp Leu Asn Asn Ala Phe Trp Thr Gln
        265                 270                 275
cag aag tac ttc acg ccg gtg ttg ggc atg ctg ttc gag tat ggc tcc 2359
Gln Lys Tyr Phe Thr Pro Val Leu Gly Met Leu Phe Glu Tyr Gly Ser
    280                 285                 290
aag ttc aag gtc gag ccg tgg gtc aag acg tgg gac cgc tgg gtg tac 2407
Lys Phe Lys Val Glu Pro Trp Val Lys Thr Trp Asp Arg Trp Val Tyr
295                 300                 305                 310
gag gac tgg ggc ggc atc tgg atc ggc cgt ctg ggc aag tac ggg gtg 2455
Glu Asp Trp Gly Gly Ile Trp Ile Gly Arg Leu Gly Lys Tyr Gly Val
                315                 320                 325
gag tcg ccg cgc agc ctc aag gac gcc aag cag gac gct tac tgg gct 2503
Glu Ser Pro Arg Ser Leu Lys Asp Ala Lys Gln Asp Ala Tyr Trp Ala
            330                 335                 340
cac cac gac ctg tat ctg ctg gct tat gcg ctg tgg ccg acc ggc ttc 2551
His His Asp Leu Tyr Leu Leu Ala Tyr Ala Leu Trp Pro Thr Gly Phe
        345                 350                 355
ttc cgt ctg gcg ctg ccg gat cag gaa gaa atg gag tgg ttc gag gcc 2599
Phe Arg Leu Ala Leu Pro Asp Gln Glu Glu Met Glu Trp Phe Glu Ala
    360                 365                 370
aac tac ccc ggc tgg tac gac cac tac ggc aag atc tac gag gaa tgg 2647
Asn Tyr Pro Gly Trp Tyr Asp His Tyr Gly Lys Ile Tyr Glu Glu Trp

375                 380                 385                 390

cgc gcc cgc ggt tgc gag gat ccg tcc tcg ggc ttc atc ccg ctg atg     2695
Arg Ala Arg Gly Cys Glu Asp Pro Ser Ser Gly Phe Ile Pro Leu Met
                395                 400                 405

tgg ttc atc gaa aac aac cat ccc atc tac atc gat cgc gtg tcg caa     2743
Trp Phe Ile Glu Asn Asn His Pro Ile Tyr Ile Asp Arg Val Ser Gln
            410                 415                 420

gtg ccg ttc tgc ccg agc ttg gcc aag ggc gcc agc acc ctg cgc gtg     2791
Val Pro Phe Cys Pro Ser Leu Ala Lys Gly Ala Ser Thr Leu Arg Val
        425                 430                 435

cac gag tac aac ggc gag atg cac acc ttc agc gac cag tgg ggc gag     2839
His Glu Tyr Asn Gly Glu Met His Thr Phe Ser Asp Gln Trp Gly Glu
    440                 445                 450

cgc atg tgg ctg gcc gag ccg gag cgc tac gag tgc cag aac atc ttc     2887
Arg Met Trp Leu Ala Glu Pro Glu Arg Tyr Glu Cys Gln Asn Ile Phe
455                 460                 465                 470

gaa cag tac gaa gga cgc gaa ctg tcg gaa gtg atc gcc gaa ctg cac     2935
Glu Gln Tyr Glu Gly Arg Glu Leu Ser Glu Val Ile Ala Glu Leu His
                475                 480                 485

ggg ctg cgc agt gat ggc aag acc ctg atc gcc cag ccg cat gtc cgt     2983
Gly Leu Arg Ser Asp Gly Lys Thr Leu Ile Ala Gln Pro His Val Arg
            490                 495                 500

ggc gac aag ctg tgg acg ttg gac gat atc aaa cgc ctg aac tgc gtc     3031
Gly Asp Lys Leu Trp Thr Leu Asp Asp Ile Lys Arg Leu Asn Cys Val
        505                 510                 515

ttc aag aac ccg gtg aag gca ttc aat tga aacgggtgtc gggctccgtc     3081
Phe Lys Asn Pro Val Lys Ala Phe Asn
    520                 525

acagggcggg gcccgacgca cgatcgttcg atcaacctca aaccaaaaag gaacatcgat 3141

atg agc atg tta gga gaa aga cgc cgc ggt ctg acc gat ccg gaa atg     3189
Met Ser Met Leu Gly Glu Arg Arg Arg Gly Leu Thr Asp Pro Glu Met
  1               5                  10                  15

gcg gcc gtc att ttg aag gcg ctt cct gaa gct ccg ctg gac ggc aac     3237
Ala Ala Val Ile Leu Lys Ala Leu Pro Glu Ala Pro Leu Asp Gly Asn
            20                  25                  30

aac aag atg ggt tat ttc gtc acc ccc cgc tgg aaa cgc ttg acg gaa     3285
Asn Lys Met Gly Tyr Phe Val Thr Pro Arg Trp Lys Arg Leu Thr Glu
        35                  40                  45

tat gaa gcc ctg acc gtt tat gcg cag ccc aac gcc gac tgg atc gcc 3333
Tyr Glu Ala Leu Thr Val Tyr Ala Gln Pro Asn Ala Asp Trp Ile Ala
50 55 60
ggc ggc ctg gac tgg ggc gac tgg acc cag aaa ttc cac ggc ggc cgc 3381
Gly Gly Leu Asp Trp Gly Asp Trp Thr Gln Lys Phe His Gly Gly Arg
65 70 75 80
cct tcc tgg ggc aac gag acc acg gag ctg cgc acc gtc gac tgg ttc 3429
Pro Ser Trp Gly Asn Glu Thr Thr Glu Leu Arg Thr Val Asp Trp Phe
85 90 95
aag cac cgt gac ccg ctc cgc cgt tgg cat gcg ccg tac gtc aag gac 3477
Lys His Arg Asp Pro Leu Arg Arg Trp His Ala Pro Tyr Val Lys Asp
100 105 110
aag gcc gag gaa tgg cgc tac acc gac cgc ttc ctg cag ggt tac tcc 3525
Lys Ala Glu Glu Trp Arg Tyr Thr Asp Arg Phe Leu Gln Gly Tyr Ser
115 120 125
gcc gac ggt cag atc cgg gcg atg aac ccg acc tgg cgg gac gag ttc 3573
Ala Asp Gly Gln Ile Arg Ala Met Asn Pro Thr Trp Arg Asp Glu Phe
130 135 140
atc aac cgg tat tgg ggc gcc ttc ctg ttc aac gaa tac gga ttg ttc 3621
Ile Asn Arg Tyr Trp Gly Ala Phe Leu Phe Asn Glu Tyr Gly Leu Phe
145 150 155 160
aac gct cat tcg cag ggc gcc cgg gag gcg ctg tcg gac gta acc cgc 3669
Asn Ala His Ser Gln Gly Ala Arg Glu Ala Leu Ser Asp Val Thr Arg
165 170 175
gtc agc ctg gct ttc tgg ggc ttc gac aag atc gac atc gcc cag atg 3717
Val Ser Leu Ala Phe Trp Gly Phe Asp Lys Ile Asp Ile Ala Gln Met
180 185 190
atc caa ctc gaa cgg ggt ttc ctc gcc aag atc gta ccc ggt ttc gac 3765
Ile Gln Leu Glu Arg Gly Phe Leu Ala Lys Ile Val Pro Gly Phe Asp
195 200 205
gag tcc aca gcg gtg ccg aag gcc gaa tgg acg aac ggg gag gtc tac 3813
Glu Ser Thr Ala Val Pro Lys Ala Glu Trp Thr Asn Gly Glu Val Tyr
210 215 220
aag agc gcc cgt ctg gcc gtg gaa ggg ctg tgg cag gag gtg ttc gac 3861
Lys Ser Ala Arg Leu Ala Val Glu Gly Leu Trp Gln Glu Val Phe Asp
225 230 235 240
tgg aac gag agc gct ttc tcg gtg cac gcc gtc tat gac gcg ctg ttc 3909
Trp Asn Glu Ser Ala Phe Ser Val His Ala Val Tyr Asp Ala Leu Phe

245 250 255
ggt cag ttc gtc cgc cgc gag ttc ttt cag cgg ctg gct ccc cgc ttc 3957
Gly Gln Phe Val Arg Arg Glu Phe Phe Gln Arg Leu Ala Pro Arg Phe
260 265 270
ggc gac aat ctg acg cca ttc ttc atc aac cag gcc cag aca tac ttc 4005
Gly Asp Asn Leu Thr Pro Phe Phe Ile Asn Gln Ala Gln Thr Tyr Phe
275 280 285
cag atc gcc aag cag ggc gta cag gat ctg tat tac aac tgt ctg ggt 4053
Gln Ile Ala Lys Gln Gly Val Gln Asp Leu Tyr Tyr Asn Cys Leu Gly
290 295 300
gac gat ccg gag ttc agc gat tac aac cgt acc gtg atg cgc aac tgg 4101
Asp Asp Pro Glu Phe Ser Asp Tyr Asn Arg Thr Val Met Arg Asn Trp
305 310 315 320
acc ggc aag tgg ctg gag ccc acg atc gcc gct ctg cgc gac ttc atg 4149
Thr Gly Lys Trp Leu Glu Pro Thr Ile Ala Ala Leu Arg Asp Phe Met
325 330 335
ggg ctg ttt gcg aag ctg ccg gcg ggc acc act gac aag gaa gaa atc 4197
Gly Leu Phe Ala Lys Leu Pro Ala Gly Thr Thr Asp Lys Glu Glu Ile
340 345 350
acc gcg tcc ctg tac cgg gtg gtc gac gac tgg atc gag gac tac gcc 4245
Thr Ala Ser Leu Tyr Arg Val Val Asp Asp Trp Ile Glu Asp Tyr Ala
355 360 365
agc gcg atc gac ttc aag gcg gac cgc gat cag atc gtt aaa gcg gtt 4293
Ser Ala Ile Asp Phe Lys Ala Asp Arg Asp Gln Ile Val Lys Ala Val
370 375 380
ctg gca gga ttg aaa taa tagaggaact attacg atg agc gta aac agc aac 4345
Leu Ala Gly Leu Lys Met Ser Val Asn Ser Asn
385 1 5
gca tac gac gcc ggc atc atg ggc ctg aaa ggc aag gac ttc gcc gat 4393
Ala Tyr Asp Ala Gly Ile Met Gly Leu Lys Gly Lys Asp Phe Ala Asp
10 15 20
cag ttc ttt gcc gac gaa aac caa gtg gtc cat gaa agc gac acg gtc 4441
Gln Phe Phe Ala Asp Glu Asn Gln Val Val His Glu Ser Asp Thr Val
25 30 35
gtt ctg gtc ctc aag aag tcg gac gag atc aat acc ttt atc gag gag 4489
Val Leu Val Leu Lys Lys Ser Asp Glu Ile Asn Thr Phe Ile Glu Glu
40 45 50
atc ctt ctg acg gac tac aag aag aac gtc aat ccg acg gta aac gtg 4537

Ile Leu Leu Thr Asp Tyr Lys Lys Asn Val Asn Pro Thr Val Asn Val
55 60 65 70
gaa gac cgc gcg ggt tac tgg tgg atc aag gcc aac ggc aag atc gag 4585
Glu Asp Arg Ala Gly Tyr Trp Trp Ile Lys Ala Asn Gly Lys Ile Glu
75 80 85
gtc gat tgc gac gag att tcc gag ctg ttg ggg cgg cag ttc aac gtc 4633
Val Asp Cys Asp Glu Ile Ser Glu Leu Leu Gly Arg Gln Phe Asn Val
90 95 100
tac gac ttc ctc gtc gac gtt tcc tcc acc atc ggc cgg gcc tat acc 4681
Tyr Asp Phe Leu Val Asp Val Ser Ser Thr Ile Gly Arg Ala Tyr Thr
105 110 115
ctg ggc aac aag ttc acc att acc agt gag ctg atg ggc ctg gac cgc 4729
Leu Gly Asn Lys Phe Thr Ile Thr Ser Glu Leu Met Gly Leu Asp Arg
120 125 130
aag ctc gaa gac tat cac gct taa ggagaatgac atg gcg aaa ctg ggt 4778
Lys Leu Glu Asp Tyr His Ala Met Ala Lys Leu Gly
135 140 1 5
ata cac agc aac gac acc cgc gac gcc tgg gtg aac aag atc gcg cag 4826
Ile His Ser Asn Asp Thr Arg Asp Ala Trp Val Asn Lys Ile Ala Gln
10 15 20
ctc aac acc ctg gaa aaa gcg gcc gag atg ctg aag cag ttc cgg atg 4874
Leu Asn Thr Leu Glu Lys Ala Ala Glu Met Leu Lys Gln Phe Arg Met
25 30 35
gac cac acc acg ccg ttc cgc aac agc tac gaa ctg gac aac gac tac 4922
Asp His Thr Thr Pro Phe Arg Asn Ser Tyr Glu Leu Asp Asn Asp Tyr
40 45 50
ctc tgg atc gag gcc aag ctc gaa gag aag gtc gcc gtc ctc aag gca 4970
Leu Trp Ile Glu Ala Lys Leu Glu Glu Lys Val Ala Val Leu Lys Ala
55 60 65
cgc gcc ttc aac gag gtg gac ttc cgt cat aag acc gct ttc ggc gag 5018
Arg Ala Phe Asn Glu Val Asp Phe Arg His Lys Thr Ala Phe Gly Glu
70 75 80 85
gat gcc aag tcc gtt ctg gac ggc acc gtc gcg aag atg aac gcg gcc 5066
Asp Ala Lys Ser Val Leu Asp Gly Thr Val Ala Lys Met Asn Ala Ala
90 95 100
aag gac aag tgg gag gcg gag aag atc cat atc ggt ttc cgc cag gcc 5114
Lys Asp Lys Trp Glu Ala Glu Lys Ile His Ile Gly Phe Arg Gln Ala
105 110 115

tac aag ccg ccg atc atg ccg gtg aac tat ttc ctg gac ggc gag cgt 5162
Tyr Lys Pro Pro Ile Met Pro Val Asn Tyr Phe Leu Asp Gly Glu Arg
120 125 130
cag ttg ggg acc cgg ctg atg gaa ctg cgc aac ctc aac tac tac gac 5210
Gln Leu Gly Thr Arg Leu Met Glu Leu Arg Asn Leu Asn Tyr Tyr Asp
135 140 145
acg ccg ctg gaa gaa ctg cgc aaa cag cgc ggt gtg cgg gtg gtg cat 5258
Thr Pro Leu Glu Glu Leu Arg Lys Gln Arg Gly Val Arg Val Val His
150 155 160 165
ctg cag tcg ccg cac tga agggaggaag tctcgccctg gacgcgacgg 5306
Leu Gln Ser Pro His
170
catcgccgtg aagtccaggg ggcagggatg ccgttccggg ccggcaggct ggcccggaat 5366
ctctggtttt cagggggcgt gccggtccac ggctcccccc ctccatcttt cgtaaggaaa 5426
tcacc atg gtc gaa tcg gca ttt cag cca ttt tcg ggc gac gca gac gaa 5476
Met Val Glu Ser Ala Phe Gln Pro Phe Ser Gly Asp Ala Asp Glu
1 5 10 15
tgg ttc gag gaa cca cgg ccc cag gcc ggt ttc ttc cct tcc gcg gac 5524
Trp Phe Glu Glu Pro Arg Pro Gln Ala Gly Phe Phe Pro Ser Ala Asp
20 25 30
tgg cat ctg ctc aaa cgg gac gag acc tac gca gcc tat gcc aag gat 5572
Trp His Leu Leu Lys Arg Asp Glu Thr Tyr Ala Ala Tyr Ala Lys Asp
35 40 45
ctc gat ttc atg tgg cgg tgg gtc atc gtc cgg gaa gaa agg atc gtc 5620
Leu Asp Phe Met Trp Arg Trp Val Ile Val Arg Glu Glu Arg Ile Val
50 55 60
cag gag ggt tgc tcg atc agc ctg gag tcg tcg atc cgc gcc gtg acg 5668
Gln Glu Gly Cys Ser Ile Ser Leu Glu Ser Ser Ile Arg Ala Val Thr
65 70 75
cac gta ctg aat tat ttt ggt atg acc gaa caa cgc gcc ccg gca gag 5716
His Val Leu Asn Tyr Phe Gly Met Thr Glu Gln Arg Ala Pro Ala Glu
80 85 90 95
gac cgg acc ggc gga gtt caa cat tga acaggtaagt tt atg cag cga gtt 5767
Asp Arg Thr Gly Gly Val Gln His Met Gln Arg Val
100 1
cac act atc acg gcg gtg acg gag gat ggc gaa tcg ctc cgc ttc gaa 5815
His Thr Ile Thr Ala Val Thr Glu Asp Gly Glu Ser Leu Arg Phe Glu
5 10 15 20

tgc cgt tcg gac gag gac gtc atc acc gcc gcc ctg cgc cag aac atc 5863
Cys Arg Ser Asp Glu Asp Val Ile Thr Ala Ala Leu Arg Gln Asn Ile
25 30 35
ttt ctg atg tcg tcc tgc cgg gag ggc ggc tgt gcg acc tgc aag gcc 5911
Phe Leu Met Ser Ser Cys Arg Glu Gly Gly Cys Ala Thr Cys Lys Ala
40 45 50
ttg tgc agc gaa ggg gac tac gac ctc aag ggc tgc agc gtt cag gcg 5959
Leu Cys Ser Glu Gly Asp Tyr Asp Leu Lys Gly Cys Ser Val Gln Ala
55 60 65
ctg ccg ccg gaa gag gag gag gaa ggg ttg gtg ttg ttg tgc cgg acc 6007
Leu Pro Pro Glu Glu Glu Glu Glu Gly Leu Val Leu Leu Cys Arg Thr
70 75 80
tac ccg aag acc gac ctg gaa atc gaa ctg ccc tat acc cat tgc cgc 6055
Tyr Pro Lys Thr Asp Leu Glu Ile Glu Leu Pro Tyr Thr His Cys Arg
85 90 95 100
atc agt ttt ggt gag gtc ggc agt ttc gag gcg gag gtc gtc ggc ctc 6103
Ile Ser Phe Gly Glu Val Gly Ser Phe Glu Ala Glu Val Val Gly Leu
105 110 115
aac tgg gtt tcg agc aac acc gtc cag ttt ctt ttg cag aag cgg ccc 6151
Asn Trp Val Ser Ser Asn Thr Val Gln Phe Leu Leu Gln Lys Arg Pro
120 125 130
gac gag tgc ggc aac cgt ggc gtg aaa ttc gaa ccc ggt cag ttc atg 6199
Asp Glu Cys Gly Asn Arg Gly Val Lys Phe Glu Pro Gly Gln Phe Met
135 140 145
gac ctg acc atc ccc ggc acc gat gtc tcc cgc tcc tac tcg ccg gcg 6247
Asp Leu Thr Ile Pro Gly Thr Asp Val Ser Arg Ser Tyr Ser Pro Ala
150 155 160
aac ctt cct aat ccc gaa ggc cgc ctg gag ttc ctg atc cgc gtg tta 6295
Asn Leu Pro Asn Pro Glu Gly Arg Leu Glu Phe Leu Ile Arg Val Leu
165 170 175 180
ccg gag gga cgg ttt tcg gac tac ctg cgc aat gac gcg cgt gtc gga 6343
Pro Glu Gly Arg Phe Ser Asp Tyr Leu Arg Asn Asp Ala Arg Val Gly
185 190 195
cag gtc ctc tcg gtc aaa ggg cca ctg ggc gtg ttc ggt ctc aag gag 6391
Gln Val Leu Ser Val Lys Gly Pro Leu Gly Val Phe Gly Leu Lys Glu
200 205 210
cgg ggc atg gcg ccg cgc tat ttc gtg gcc ggc ggc acc ggg ttg gcg 6439
Arg Gly Met Ala Pro Arg Tyr Phe Val Ala Gly Gly Thr Gly Leu Ala

215 220 225
ccg gtg gtc tcg atg gtg cgg cag atg cag gag tgg acc gcg ccg aac 6487
Pro Val Val Ser Met Val Arg Gln Met Gln Glu Trp Thr Ala Pro Asn
230 235 240
gag acc cgc atc tat ttc ggt gtg aac acc gag ccg gaa ttg ttc tac 6535
Glu Thr Arg Ile Tyr Phe Gly Val Asn Thr Glu Pro Glu Leu Phe Tyr
245 250 255 260
atc gac gag ctc aaa tcc ctg gaa cga tcg atg cgc aat ctc acc gtg 6583
Ile Asp Glu Leu Lys Ser Leu Glu Arg Ser Met Arg Asn Leu Thr Val
265 270 275
aag gcc tgt gtc tgg cac ccg agc ggg gac tgg gaa ggc gag cag ggc 6631
Lys Ala Cys Val Trp His Pro Ser Gly Asp Trp Glu Gly Glu Gln Gly
280 285 290
tcg ccc atc gat gcg ttg cgg gaa gac ctg gag tcc tcc gac gcc aac 6679
Ser Pro Ile Asp Ala Leu Arg Glu Asp Leu Glu Ser Ser Asp Ala Asn
295 300 305
ccg gac att tat ttg tgc ggt ccg ccg ggc atg atc gat gcc gcc tgc 6727
Pro Asp Ile Tyr Leu Cys Gly Pro Pro Gly Met Ile Asp Ala Ala Cys
310 315 320
gag ctg gta cgc agc cgc ggt atc ccc ggc gaa cag gtc ttc ttc gaa 6775
Glu Leu Val Arg Ser Arg Gly Ile Pro Gly Glu Gln Val Phe Phe Glu
325 330 335 340
aaa ttc ctg ccg tcc ggg gcg gcc tga accggggaag taccgtgacc 6822
Lys Phe Leu Pro Ser Gly Ala Ala
345
accgagcagt tcccgcccca attcctgcgt gaaatgatcg agcagctgga cgccagcatc 6882
caggagctcg cacgcaagga aaagggactt gcggcatccc tgggcacggg ccgggtcgcc 6942
gagctcaagg aatactggga ccacgttctc actcccgagg aggaatggga gctcaagcga 7002
ccatggactt ccgcgaccgg gaactggtgt ggatctggtc ccgtctcagg cgggcccgaa 7062
cctcccgcgc caatgccggg gaggcctata tcgccagctg tcgccggcgc cgcgaaaaaa 7122
cgaacaatcc tgaaacggag tcgacgggta tcgttgggaa aaaggttatc tgtcgcccta 7182
ttttgt 7188